# EUROPEAN PATENT SPECIFICATION

(11) **EP 1 148 882 B1**
(45) Date of publication and mention of the grant of the patent: **01.08.2007**
(21) Application number: 00903916.5
(22) Date of filing: 28.01.2000
(51) Int. Cl.: A61K 31/519, A61K 31/57, A61K 31/4415, A61P 7/02

(54) **USE OF A PLASMA HOMOCYSTEINE CONTENT REDUCING AGENT FOR THE REDUCTION OF THE THROMBOEMBOLIC SIDE EFFECT RISK INDUCED BY GESTAGEN TYPE HORMONES**
VERWENDUNG EINES DEN PLASMA HOMOCYSTEINGEHALT REDUZIERENDEN WIRKSTOFFS ZUR VERMINDERUNG DES RISIKOS DER DURCH GESTAGEN HORMONE VERURSACHTEN THROMBOEMBOLISCHEN NEBENWIRKUNGEN
UTILISATION D'UN AGENT REDUISANT LA TENEUR EN HOMOCYSTEINE DANS LE PLASMA POUR LA REDUCTION DU RISQUE D'EFFETS SECONDAIRES THROMBOEMBOLIQUES INDUITS PAR LES HORMONES GESTAGENES

(30) Priority: 01.02.1999 HU 9900213
(43) Date of publication of application: 31.10.2001
(73) Proprietor: Bogye, Gabor, 1027 Budapest (HU)
(72) Inventor: Bogye, Gabor, 1027 Budapest (HU)
(74) Representative: Beetz & Partner
(86) International application number: PCT/HU2000/000009
(87) International publication number: WO 2000/044385

(56) References cited:
- WO-A-00/38691
- WO-A-00/51596
- GB-A- 2 131 292
- BARNABEI V.M. ET AL: "Plasma homocysteine in women taking hormone replacement therapy: The postmenopausal Estrogen/Progestin Interventions (PEPI) trial." JOURNAL OF WOMEN'S HEALTH AND GENDER-BASED MEDICINE, (1999) 8/9 (1167-1172). , XP000913564
- RHODE ET AL: "Effect of Orange Juice, Folic acid, and Oral Contraceptives on Serum Folate in Women taking a Folate-Restricted Diet" JOURNAL OF THE AMERICAN COLLEGE OF NUTRITION,US,AMERICAN COLLEGE OF NUTRION, WILMINGTON, NC, vol. 2, no. 3, 1983, pages 221-230-230, XP002114784 ISSN: 0731-5724
- BUTTERWORTH ET AL: "Improvement in Cervical Dysplasia Associated with Folic Acid Therapy in Users of Oral Contraceptives" AMERICAN JOURNAL OF CLINICAL NUTRITION,US,BETHESDA,MD, vol. 35, no. 1, January 1982 (1982-01), pages 73-82-82, XP002114785
- WHITEHEAD ET AL: "Megaloblastic Changes in the Cervical Epithelium. Association with Oral Contraceptive Therapy and Reversal with Folic acid" JAMA THE JOURNAL OF THE AMERICAN MEDICAL ASSOCIATION,US,CHICAGO,IL, vol. 226, no. 12, 17 December 1973 (1973-12-17), pages 1421-1424-1424, XP002114786 ISSN: 0098-7484

## Description

The present invention relates to the use of compounds lowering the plasma level of homocysteine in humans for the manufacture of medicaments for lowering the risk of thromboembolic side effects of gestagen type steroid hormone compositions.

It has been known that the most important side effect of the use of some compositions containing steroid hormones, such as gestagens, is the increased occurrence of thromboembolic diseases. These side effects may occur upon the administration of any pharmaceutical composition comprising gestagen type hormone, and they are often lethal.

This problem has been solved so far by two methods:
a) Patients, in case of which the probability of the occurrence of thromboembolic side effects is rather high (adipose, smokers, patients of the age above 35, patients whose anamnesis already showed thromboembolic disease), were excluded from this hormone therapy.
b) On the basis of the positive correlation of the occurrence of the thromboembolic complications and the dose of the used hormone, the occurrence of the thromboembolic complications could be reduced by decreasing the hormone content of the pharmaceutical compositions.

These known methods show numerous disadvantages:

The patients excluded from the hormone therapy on the basis of the absolute and relative contraindications could not obtain an otherwise necessary treatment.

By reducing the hormone content of the given pharmaceutical composition, not only the occurrence of the side effects is reduced, but the extent and safety of the effect to be achieved, too.

A further disadvantage of this latter known method is that although the risk of the occurrence of the side effects is reduced, the potentially lethal side effects cannot be entirely eliminated.

On the basis of recent research it has become known, that the increase of the homocysteine content in the human plasma is an independent risk factor for arterial and venal thrombosis and embolism. Reference is made to recent studies summarising this problem: Welch, G.N., Loscalzo, J., Homocysteine and atherothrombosis, N. Engl. J. Med. 338 (1998) 1042-1050; den Heijer, M., Kostor, T., Blom, H.J., et al,. Hyperhomocysteinemia as a risk factor for deep-vein thrombosis, N. Engl. J. Med. 334 (1996) 759-762; Graham, I.M., Daly, I.E., Refsum, H., et al., Plasma homocysteine as a risk factor for vascular disease, JAMA, 277 (1997) 1775-1781).

EP 0 347 864 A2 discloses that atherosclerosis can be reduced by using some pharmaceutical compositions reducing the homocysteine content of the plasma and/or of the arterial walls.

Some compounds (folic acid, vitamin B₆, vitamin B₁₂, betaine, choline, acetyl cysteine) have been known to reduce the homocysteine content of the plasma in cases of certain types of hyperhomocysteinaemia (not all types), cf. Welch, G.N., Loscalzo, J., Homocysteine and atherothrombosis, N. Engl. J. Med. 338 (1998) 1042-1050; Refsum, H., Ueland, P.M., Clinical significance of pharmacological modulation of homocysteine metabolism, TiPS 11 (1990) 411-416).

In the case of some of these B vitamins (folic acid, vitamins B₆ and B₁₂), it can be considered as proven that they reduce the occurrence of thromboembolic complications not induced by medicines, cf. Graham, I.M., Daly, I.E., Refsum, H., et al., Plasma homocysteine as a risk factor for vascular disease, JAMA 277 (1997) 1775-1781; Herbert, V., Bigaouette, J., Call for endorsement of a Petition to the Food and Drug Administration to always add vitamin B12 to any folate fortification or supplement, Am. J. Clin. Nutr. 65 (1997) 572-573).

In the case of steroid hormones containing estrogen, it has been already suggested that these hormones tend to make susceptible to thromboembolism through the increase of the homocysteine content of the plasma, but this hypothesis could not be proved by tests carried out so far (Brattstrom, L., Israelsson, B., Olsson, A., Andersson, A., Hultberg, B., Plasma homocysteine in women on oral oestrogen-containing contraceptives and in men with oestrogen-treated prostatic carcinoma, Scand. J. Clin. Invest., 52 (1992) 283-287).

The publication of Whitehead, N., Reyner, F., and Lindenbaum, J., Megaloblastic Changes in the Cervical Epithelium, JAMA 226 (1973) 1421-1424, relates to a study showing that in women taking oral contraceptives (combination type or progesterone only), the treatment with folic acid reverted to normal or improved megaloblastic abnormalities of cervicovaginal cells similar to those seen in severe folate and vitamin B₁₂ deficiency. In this study, 17 - 21 % of the women had decreased serum folate concentration. This publication does not refer to the reduction of homocysteine plasma levels nor the risk of thromboembolic side effects induced by gestagen hormones.

WO 00/38691, which represents a prior art document pursuant to Art. 54(3) and (4) EPC, discloses combinations of an estrogen and a progestogen for oral contraception which may comprise nutritional supplements such as folic acid.

Thus, there was a continuing need for a medicament the use of which results in a lower risk of thromboembolic diseases while maintaining the original activity of the hormone(s).

It has now surprisingly been found that the occurrence of thromboembolic diseases which can be correlated with the administration of pharmaceutical compositions containing gestagen type steroid hormones, is greatly due to the plasma homocysteine level increasing activity of the gestagen hormones. It has further been recognised that this increase of the plasma homocysteine level caused by gestagen hormones can efficiently be reduced or prevented by known homocysteine level reducing agents, e.g. folic acid, vitamin B₆, vitamin B₁₂, betaine, choline and acetyl cysteine, which have not been used for this purpose so far.

Thus, in view of the above-indicated prior art, it is the underlying problem of the present invention to provide a medicament for the reduction of thromboembolic side effects induced by gestagen type hormone compositions.

The above problem is solved according to claim 1 by the use of one or more plasma homocysteine content reducing agents for the manufacture of medicaments for the reduction of the thromboembolic side effect risk induced by gestagen type hormone containing compositions.

The dependent claims relate to preferred embodiments.

Preferably, the plasma homocysteine content reducing agent is selected from folic acid, vitamin B₆, vitamin B₁₂, betaine, choline and acetyl cysteine, folic acid and vitamin B₆ being particularly preferred.

The pharmaceutical composition(s) obtained according to the use in accordance with the present invention comprise an efficient amount of plasma homocysteine level reducing compound(s), e.g. folic acid, vitamin B₆, vitamin B₁₂, betaine, choline, acetyl cysteine, and may further comprise an efficient amount of gestagen type steroid hormone(s) (progesterone type hormone(s)).

It is a favourable feature of such pharmaceutical compositions that the hormone component(s) thereof is (are) suitable for contraception, hormone substituting therapy, antiinflammation, promoting in vitro fertilisation, dermatological therapy and/or cosmetological treatment, and the compositions may be used for these purposes.

The hormone component and the homocysteine level reducing component may be administered separately, but it is preferred to administer the active components in the form of one composition.

On the basis of the above recognition, the following unexpected results have been achieved:
- The pharmaceutical compositions containing gestagen type steroid hormones increase the homocysteine concentration of the plasma, meaning the increase of the risk of thromboembolic diseases.
- The increased plasma homocysteine level due to a medicine containing gestagen type hormone can especially efficiently be reduced by folic acid and vitamin B₆. We note that an the basis of data having been available to us before our investigations, one could not come to the conclusion that B vitamins would be efficient against hyperhomocysteinaemia induced by medicines comprising gestagen type hormones, as folic acid and vitamin B₆ are not efficient in all types of hyperhomocysteinaemia, (Hong, S.Y., Yang; D.H., Chang, S.K., Plasma homocysteine, vitamin B6, vitamin B12 and folic acid in end-stage renal disease during low-dose supplementation with folic acid, Am. J. Nephrol., 18 (1998) 367-372).
- In hyperhomocysteinaemia induced by medicines comprising gestagen type hormones the folic acid and vitamin B₆ therapy results in a much more intensive plasma homocysteine level reduction than it could have been expected on the basis of the prior art. According to the prior art in case of folic acid therapy against folic acid deficiency, the plasma homocysteine concentration is diminished on an average by 45 to 50% and in case of hyperhomocysteinaemia of genetic origin on an average by 20 to 25 % (Guttormsen, A.B., Schneede, J., Ueland, P.M., Refsum, H., Kinetics of total plasma homocysteine in subject with hyperhomocysteinemia due to folate or cobalamin deficiency, Am. J. Clin. Nutr. 63 (1996) 194-202).

The advantage of the pharmaceutical composition(s) used according to the invention as compared to compositions containing only hormone is the smaller risk of thromboembolic side effects. The use of gestagen type hormones becomes safer by the medicaments used according to the invention, and one can expect that for patient groups (smokers, patients above the age of 35, patients suffering from overweight, in case of anamnestic data, etc.), who were excluded from the use of compositions containing solely hormone, said pharmaceutical composition(s) can be prescribed. A further advantage is that both the hormone(s) and the compounds reducing the plasma homocysteinaemia concentration can be finished in one dosage unit (tablets, capsules, ampoules, powders, solutions, granules, syrups, etc.) ensuring thereby the simultaneous application of the hormone active ingredient and the "antidote" acting against the most important side effect of the hormone, i.e., the plasma homocysteine reducing agent.

The hormone component(s) and the plasma homocysteine level reducing compound(s) may be applied in separate dosage units as well, as according to our test results both in case of simultaneous and separate administration, hyperhomocysteinaemia induced by gestagen hormones may be reduced or prevented.

For treating patients, taking medicines containing gestagen type hormone(s), a medicament obtained according to the invention comprising an effective dosage of plasma homocysteine content reducing agents, preferably selected from folic acid, vitamin B₆, vitamin B₁₂, betaine, choline and acetyl cysteine, is administered.

The effective dosage is generally in the range of from 100 µg to 9 g per day of plasma homocysteine content reducing agents, ranging, for example, from 0.5 to 5 mg per day for folic acid, from 10 to 300 mg for vitamin B₆, from 300 µg to 5 mg per day for vitamin B₁₂, from 0.5 to 9 g per day for betaine and from 100 mg to 1 g per day for acetyl cysteine.

The effective dosage of gestagen components is generally in the range of 0.05 to 5 mg per day.

The invention will be further explained by the following non-limiting examples:

### Example 1

47 healthy women of middle age were involved in the test. They were divided in two groups on the basis of taking or not taking contraceptives, and then their fasting plasma homocysteine concentration was measured by the HPLC method. The average homocysteine content of the women obtaining entirely or partially gestagen containing contraceptive therapy (daily 1) 0.15 mg of levonorgestrelum and 0.03 mg of aethinyloestradiolum, or 2) 0.25 mg of levonorgestrelum and 0.05 mg of aethinyloestradiolum, or 3) 0.15 mg of desogestrelum and 0.03 mg of aethinyloestradiolum, or 4) 0.075 mg of gestodenum and 0.03 mg of aethinyloestradiolum, or 5) 0.25 mg of norgestinatum and 0.035 mg of aethinyloestradiolum, or 6) 0.5 mg of aethynodiolum diaceticum) was significantly higher than that of the control group.

### Example 2

32 healthy women of middle age were involved in the test. They were taking oral contraceptive of gestagen content (daily 1) 0.15 mg of levonorgestrelum and 0.03 mg of aethinyloestradiolum, or 2) 0.25 mg of levonorgestrelum and 0.05 mg of aethinyloestradiolum, or 3) 0.15 mg of desogestrelum and 0.03 mg of aethinyloestradiolum, or 4) 0.075 mg of gestodenum and 0.03 mg of aethinyloestradiolum, or 5) 0.25 mg of norgestinatum and 0.035 mg of aethinyloestradiolum, or 6) 0.5 mg of aethynodiolum diaceticum). The fasting plasma homocysteine content was measured by HPLC. It was found that the extent of increase of the plasma homocysteine content was in correlation with the daily dosage of gestagen. It was also noticed an increase of the plasma homocysteine content, when an exclusively gestagen containing composition was administered to the patients. It is apparent that gestagens are responsible for the increase of the homocysteine concentration.

### Example 3

31 healthy women of middle age were examined. They obtained, in addition to a gestagen containing contraceptive (daily 1) 0.15 mg of levonorgestrelum and 0.03 mg of aethinyloestradiolum, or 2) 0.25 mg of levonorgestrelum and 0.05 mg of aethinyloestradiolum, or 3) 0.15 mg of desogestrelum and 0.03 mg of aethinyloestradiolum, or 4) 0.075 mg of gestodenum and 0.03 mg of aethinyloestradiolum, or 5) 0.25 mg of norgestinatum and 0.035 mg of aethinyloestradiolum, or 6) 0.5 mg of aethynodiolum diaceticum), 1 or 3 mg/die of folic acid or 20 mg/die of vitamin B₆. When the fasting plasma homocysteine concentration was measured by HPLC, a significant (p<0.05) reduction (on an average by 69%) was observed. Some participants (n= 14) obtained separately vitamin tablets in addition to the contraceptive tablets, and in other cases (n= 17), the contraceptive and the folic acid or vitamin B₆ were ground to a powder, admixed and filled into cachets or capsules and the mixture thus obtained was administered. As to the plasma homocysteine concentration, no difference could be shown between the two methods of administration (in separate tablets or one single formulation). During the observation period no undesired pregnancy or thromboembolic complication occurred.

### Example 4

In the case of 6 women during a gestagen hormone therapy, which was used in an in vitro fertilisation program (the dosage of 600 mg/die of progesterone was gradually reduced during 12 weeks), the change of fasting plasma homocysteine concentration was measured by HPLC. The result was that the plasma homocysteine concentration changed proportionately with the dosage of gestagen.

### Example 5

During taking gestagen containing hormone (600 mg/die of progesterone) the fasting plasma homocysteine concentration was measured, and the therapy was then supplemented with 1 mg/ die and 3 mg/die of folic acid or 20 mg/die of vitamin B₆ by placing the hormone in the form of micronised progesterone and the vitamin B in the form of folic acid or vitamin B₆ into a capsule or cachet, which was administered. The plasma homocysteine concentration was reduced on an average by 65%.

The pharmaceutical compositions according to the use of the invention may be prepared by methods known per se. Depending on the route of administration one can prepare tablets, vaginal tablets, capsules, granules, cachets, syrups, solutions, dragees, suppositories, ampoules, etc.

Accordingly, the conventionally used solid or liquid carriers can be added to the active ingredients. As solid carrier one may use aroma substances, lubricating agents, solubilisers, suspending agents, gliding agents, disintegrating agents, tabletting or capsulating agents, such as magnesium stearate, talc, methyl cellulose, gelatine, sodium carboxymethyl cellulose, polyvinyl pyrrolidone, lactose, etc.

As liquid carriers one may use e.g. buffers, preservatives, agents controlling viscosity or osmotic pressure, emulsifiers, solubilisers, sweetening agents, such as water, cellulose derivatives, alcohols, oils or oil esters, etc.

Hereinbelow, a short description of the preparation of compositions containing the hormone and the compound reducing the plasma homocysteine concentration will be given:

### Example 6

Tablets containing 1) 0.15 mg of levonorgestrelum and 0.03 mg of aethinyloestradiolum, or 2) 0.25 mg of levonorgestrelum and 0.05 mg of aethinyloestradiolum, or 3) 0.15 mg of desogestrelum and 0.03 mg of aethinyloestradiolum, or 4) 0.075 mg of gestodenum and 0.03 mg of aethinyloestradiolum, or 5) 0.25 mg of norgestinatum and 0.035 mg of aethinyloestradiolum, or 6) 0.5 mg of aethynodiolum diaceticum were ground to powder, whereafter tablets containing 1 mg or 3 mg of folic acid or 20 mg of vitamin B₆ were added in the form of powder. The mixture was filled into cachets or hard gelatine capsules.

### Example 7

Soft gelatine capsules containing 500 mg of micronised progesterone were opened, and tablets containing 3 mg of folic acid or 20 mg of vitamin B₆ were ground to powder. The oil content of the progesterone tablets was absorbed by the powder of the vitamin tablets, and the mixture was filled into hard gelatine capsules, sealed or packed into cachets.

## Claims

1. Use of one or more plasma homocysteine content reducing agents for the manufacture of medicaments for the reduction of the thromboembolic side effect risk induced by gestagen type hormone containing compositions.

2. Use according to claim 1, wherein the plasma homocysteine content reducing agent is selected from folic acid, vitamin B₆, vitamin B₁₂, betaine, choline and acetyl cysteine.

3. Use according to claim 2, wherein folic acid is used.

4. Use according to claim 2, wherein vitamin B₆ is used.

5. Use according to any of claims 1 to 4, wherein the manufactured medicament comprises at least one gestagen type hormone.

6. Use according to claim 5, wherein the manufactured medicament comprises progesterone.

## Patentansprüche

1. Verwendung eines oder mehrerer den Plasma-Homocysteingehalt reduzierender Wirkstoffe zur Herstellung von Arzneimitteln zur Verminderung des Risikos von thromboembolischen Nebenwirkungen, die durch Hormone vom Gestagen-Typ enthaltende Zusammensetzungen hervorgerufen werden.

2. Verwendung nach Anspruch 1, wobei der den Plasma-Homocysteingehalt reduzierende Wirkstoff ausgewählt ist unter Folsäure, Vitamin B₆, Vitamin B₁₂, Betain, Cholin und Acetylcystein.

3. Verwendung nach Anspruch 2, wobei Folsäure verwendet wird.

4. Verwendung nach Anspruch 2, wobei Vitamin B₆ verwendet wird.

5. Verwendung nach einem der Ansprüche 1 bis 4, wobei das hergestellte Arzneimittel mindestens ein Hormon vom Gestagen-Typ enthält.

6. Verwendung nach Anspruch 5, wobei das hergestellte Arzneimittel Progesteron enthält.

## Revendications

1. Utilisation d'un ou plusieurs agents réduisant la teneur en homocystéine dans le plasma pour la fabrication de médicaments pour réduire le risque d'effets secondaires thromboemboliques induits par les compositions contenant des hormones du type des gestagènes.

2. Utilisation selon la revendication 1, dans laquelle l'agent reduisant la teneur en homocysteine dans le plasma est choisi parmi l'acide folique, la vitamine B₆, la vitamine B₁₂, la bétaïne, la choline et l'acétyle cystéine.

3. Utilisation selon la revendication 2, dans laquelle l'acide folique est utilisé.

4. Utilisation selon la revendication 2, dans laquelle la vitamine B₆ est utilisée.

5. Utilisation selon l'une quelconque des revendications 1 à 4, dans laquelle le médicament fabriqué contient au moins une hormone du type des gestagènes.

6. Utilisation selon la revendication 5, dans laquelle le médicament fabriqué contient de la progestérone.
